Europäisches Patentamt

European Patent Office

Office européen des brevets

Publication number: **0 319 613**

**A1**

# EUROPEAN PATENT APPLICATION

(21) Application number: 87202433.6

(22) Date of filing: 07.12.87

(51) Int. Cl.4 **C08F 8/28** , **C08F 8/12** , **C07C 47/04**

(43) Date of publication of application:
**14.06.89 Bulletin 89/24**

(84) Designated Contracting States:
**AT BE CH DE ES FR GB GR IT LI LU NL SE**

(71) Applicant: **METHANOL CHEMIE NEDERLAND V.o.F.**
**Rijksstraatweg 32**
**NL-9752 AE Haren(NL)**

(72) Inventor: **Roerdink, Eize**
**Laathofstraat 15**
**NL-6191 GD Beek (L)(NL)**
Inventor: **Bruls, Wilhelmus Gerardus Marie**
**Graaf Wolterhoenstraat 32**
**NL-6243 BE Meerssen(NL)**
Inventor: **Steeman, Reinard Jozef Maria**
**J. Evertsenstraat 31**
**NL-6181 DD Stein(NL)**

(74) Representative: **Hoogstraten, Willem Cornelis Roeland et al**
**OCTROOIBUREAU DSM Postbus 9**
**NL-6160 MA Geleen(NL)**

(54) **A process for preparing polymers and their use as stabilizers for aqueous formaldehyde solutions.**

(57) The present application is concerned with a process for preparing polymers comprising vinyl acetate-, vinyl alcohol- and vinyl acetal-units by alcoholyzing and acetalyzing a polyvinyl acetate having a relatively low molecular weight in a solution in an organic solvent having a high polyvinyl acetate concentration in the presence of a strong acid catalyst at elevated temperatures, preferably 40 to 65°C, adding an alkaline agent after completion of the alcoholysis and acetalyzation to the desired degree to neutralize the acid catalyst, separating the salt formed on neutralization and recovering the produced polymer. This process is characterized by the fact that the separation of the formed salt is carried out by sedimentation.

By this process it is possible to separate very easily the produced polymer from the salt nearly quantitatively.

# A process for preparing polymers and their use as stabilizers for aqueous formaldehyde solutions

The present invention is concerned with a process for preparing polymers which can be used as stabilizers for aqueous formaldehyde solutions.

US-patent 4 085 079 is concerned with a method of stabilizing concentrated aqueous formaldehyde solutions by the addition of a stabilizing amount of a polymer of vinyl acetate-, vinyl acetal- and vinyl aclohol-units to the formaldehyde solution.

The mentioned polymers are prepared from a relatively low molecular weight polyvinyl acetate of weight average molecular weight in the range of from about 2000 to about 60000 and preferably in the range of from about 6000 to about 20000 by dissolving them in a low boiling alcohol selected from the group consisting of methanol, ethanol, propanol and iso propyl alcohol and subjecting the obtained solution to an alcoholysis and acetalyzation. For this purpose acetaldehyde is added and the alcoholysis and acetalyzation is carried out in the presence of a strong acid catalyst. The amounts of alcohol and acetaldehyde, the ratio of alcohol to acetaldehyde and the time and temperature of the alcoholysis and acetalyzation reaction are selected dependent on the desired ratio of vinyl acetate-, vinyl acetal- and vinyl alcohol-units within the polymer. The alcoholysis and acetalyzation can be carried out concurrently or sequentially. The acid catalyst can be any acid with a pKa at 25°C of at least about 2,0, preferably strong mineral acids. as sulfuric acid and hydrochloric acid, are used.

When the desired degree of alcoholysis and acetalyzation has been achieved the reaction solution is treated with an alkaline agent, as alkali or alkaline earth metal oxide, hydroxide, carbonate, bicarbonate or salt of a low boiling organic acid to neutralize the strong acid catalyst. Sodium propionate and potassium acetate are the preferred neutralizing agents. The polymer solution is than dried to remove the alcohol solvent, is washed repeatedly with water to remove inorganic salts and is again dried. However, since the produced polymer is insoluble in water it precipitates on contact with water and forms lumps occluding the salt particles formed on neutralizsation which only difficultly can be separated from the polymer lumps. This means that if the polymer is redissolved in a solvent to be added to the formaldehyde solution to be stabilized a considerable amount of salt is transferred into the formaldehyde solution. Naturally, this is not desired.

The process of the mentioned US-patent 4 085 079 is starting from a polyvinyl acetate solution having a high polyvinyl acetate concentration in the order of 60 to 70 % by weight. A similar process is described in the US-patent 4 247 487, however, this process is starting from a polyvinyl acetate solution having a low polyvinyl acetate concentration in the order of 5 % by weight. The use of such a low polyvinyl acetate concentration solution has the drawback that a considerable amount of the strongacid catalyst has to be used, and this necessitates the use of a high amount of the alkaline agent to neutralize the acid. This in turn produces a high amount of salt formed on neutralization. Therefore, according to this process the ratio of salt to polymer is very high and varies between a range of 2:1 and 3:1. Obviously, such a high amount of salt is difficultly to separate from the reaction mixture. A filtration is no suitable means for removing the salt because the salt is precipitated in form of very small particles which are difficultly to filtrate. This might be the reason why in the US-patent 4 085 079 contrary to the US-patent 4 247 487 no filtration but only the washing step is mentioned to remove the salt.

Object of the present application is, therefore, the provision of a process of the type described in the US-patent 4 085 079, however, without the above outlined drawbacks resulting from washing out the formed salt by water.

The invention is basing on the discovery that sedimentation is a very effective and easily to be carried out means for separating the salt formed on neutralization of the acid catalyst with an alkaline agent.

Accordingly, the present application is concerned with a process for preparing polymers comprising vinyl acetate-, vinyl alcohol- and vinyl acetal-units by alcoholyzing and acetalyzing a polyvinyl acetate having a relatively low molecular weight in a solution in an organic solvent having a high polyvinyl acetate concentration in the presence of a strong acid catalyst at eluated temperatures, preferably 40 to 65°C, adding an alkaline agent after completion of the alcoholysis and acetalyzation to the desired degree to neutralize the acid catalyst, separating the salt formed on neutralization and recovering the produced polymer. This process is characterized by the fact that the separation of the formed salt is carried out by sedimentation.

Therefore, the present invention involves a process of the type as described in the US-patent 4 085 079 or similar processes with the exception that the salts formed on neutralizing the strong acid catalyst with an alkaline agent are separated by sedimentation.

Preferably, the process of the present invention is carried out to produce quaterpolymers according to the co-pending patent application (patent application which has been filed at the same date like the present application under the internal file number 6007). These quaterpolymers are very well suited as stabilizers of aqueous formaldehyde solutions because of their high solubility in formaldehyde in comparison to any known prior art stabilizers comprising vinyl acetate-, vinyl alcohol- and vinyl acetal-units.

As already mentioned, the process of the present application can be carried out in the same manner as the above outlined known processes up to the step of separating the salt formed on neutralization of the acid catalyst with an alkaline agent. According to the present invention the reaction mixture containing the precipitated salt is introduced in one or more sedimentation vessels, especially sedimentation columns, and is subjected to a sedimentation which normally proceeds with a migration velocity of the boundery layer between supernatant and salt particles containing liquid of approximately 6 to 8 mm per hour.

The sedimentation can be accelerated by centrifuging the mixture withdrawn from the reaction vessel where the alcoholysis and acetalyzation have been carried out. The centrifugation can be carried out to promote the sedimentation, it also can be carried out after the sedimentation is more or less completed.

Additionally, the supernatant obtained after sedimentation and sedimentation/centrifugation, respectively can be filtered by suitable means to remove the last traces of fine particulate solids.

According to another preferred embodiment the reaction mixture after the reaction has proceeded for a certain period of time can be stripped to remove any excess of acetaldehyde, preferably by using an inert gas like nitrogen which is introduced into the optionally stirred reaction mixture underneath the liquid surface. This stripping preferably is carried out when the alcoholysis and acetalyzation are carried out over a long period of time as disclosed in the mentioned co-pending application to produce the specific quaterpolymers disclosed in this application. If for example the alcoholysis and acetalyzation are carried out for a period of 6 hours the stripping may be carried out 2 hours after start of the acetalyzation. A stripping period of 5 to 20 minutes, especially 10 minutes, is normally sufficient.

The polymers prepared according to the process of the present invention can be used as stabilizers for aqueous formaldehyde solutions in a manner as for example disclosed in the US-patent 4 085 079 or in the above mentioned co-pending application of the same applicants.

The following example is set forth to illustrate the invention but is not to be construed as limiting the scope thereof.

## Example 1

Into a reaction vessel purged with nitrogen and containing 300 g methanol having a water content of less than 0,3 % by weight 667 g polyvinyl acetate having an average molecular weight of approximately 15000 (VINNAPAS B 1,5, Wacker-Chemie) are added and dissolved in the methanol during 2,5 hours under slow stirring at room temperature. After the dissolution has been completed the obtained solution is stirred to obtain a homogeneous solution.

To this solution 200 g acetaldehyde are added to the closed reaction vessel.

Then 61,8 g sulfuric acid (96 %) (0,605 mole) in 53,4 g methanol are added to the closed reactor.

The reaction mixture is now heated to 60 ± 1°C, and the acetalyzation is carried out for a period of 5 hours.

After a cooling of the reaction mixture 118,6 g potassium acetate in 560 g methanol are pumped into the reactor.

By this procedure a quaterpolymer containing 6,8 mole percent hemiacetal-units and 51,2 % acetal-units apart from the vinyl acetate- and vinyl aclohol-units is obtained. The solution has a total solids content of 30,6 % by weight.

The obtained reaction mixture (1978 g) with a theoretical quaterpolymer amount of 650 g is subsequently submitted to sedimentation over night in a sedimentation column.

The supernatant consists of 936 g of a clear solution with a total solids content of 29,7 % by weight resulting in a recovery of 278 g quaterpolymer.

To the remaining suspension of salt consisting mainly of potassium sulfate an additional amount of 400 g methanol is added. After stirring and sedimentation overnight 86 g of a clear solution are separated as supernatant having a total solids content of 20,9 % by weight resulting in a recovery of an additional 182 g of quaterpolymer.

To the remaining suspension an additional amount of 200 g methanol is added. After stirring and sedimention overnight 320 g of a clear solution are separated as supernatant with a total solids content of 16,2 % by weight resulting in a recovery of additional 52 g of the quaterpolymer.

Therefore, the total recovery amounts to 512 g quaterpolymer corresponding to a yield of 84 %.

Therefore, this example shows that the separation of the quaterpolymer from the salt can be achieved nearly quantitatively by simple sedimentation.

Example 2

Into a reactor 1.35 parts by weight methanol are introduced. Then 245 parts by weight polyvinyl acetate having a weight average molecular weight of 15000 are dissolved in the methanol under stirring until the dissolution is completed. Under cooling 0.23 parts by weight sulfuric acid (98 % by weight $H_2SO_4$) are dissolved in 0.2 parts by weight methanol. During the dissolution the temperature is maintained below 35°C.

Thereupon 0.73 parts by weight acetaldehyde are added to the reactor. Subsequently the sulfuric acid methanol-mixture is added to the reactor. The reactor content is heated to 60°C. During 2 hours the reactor content is maintained at 60°C while it is intensively stirred. Next the reactor content is stripped by means of nitrogen (15 l $N_2$/l reactor volume) during about 15 minutes. During stripping the reaction temperature is maintained constant. After stripping the reaction is allowed to continue for 4 hours at 60°C. The reaction is stopped by adding to the reactor a solution of 0,44 parts by weight potassium acetate in 2,13 parts by weight methanol.

During the neutralization with potassium acetate a very finely divided precipitate of potassium sulfate is formed (particle size < 3 μm). This salt cannot be separated from the reaction mixture by filtration. The whole reaction mixture is transferred to a sedimentation column in which the separation of the salt from the reaction product takes place by sedimentation under influence of gravity. It has been found that with a liquid height of 0,5 to 0,6 m a good separation can be obtained in 4 days when a sedimentation column having a H/D-ratio of 1 is used. The clear supernatant is drawn off after 4 days and filtered to remove any suspended impurities. After dilution the solution is ready for use.

## Claims

1. A process for preparing polymers comprising vinyl acetate-, vinyl alcohol- and vinyl acetal-units by alcoholyzing and acetalyzing a polyvinyl acetate having a relatively low molecular weight in a solution in an organic solvent having a high polyvinyl acetate concentration in the presence of a strong acid catalyst at elevated temperatures, preferably 40 to 65°C, adding an alkaline agent after completion of the alcoholysis and acetalyzation to the desired degree to neutralize the acid catalyst, separating the salt formed on neutralization and recovering the produced polymer, characterized in that the separation of the formed salt is carried out by sedimentation.

2. A process according to claim 1, characterized in that the sedimentation is promoted by centrifugation.

3. A process according to claim 1 and 2, characterized in that prior to the completion of the alcoholysis and acetalyzation step the reaction mixture is stripped, preferably by introducing an inert gas into the reaction mixture.

4. A process according to the claims 1 to 3, characterized in that the supernatant obtained after sedimentation is filtered.

5. The use of the polymers obtained according to the claims 1 to 4 as stabilizers for aqueous formaldehyde solutions.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 105, no. 26, December 1986, page 20, abstract no. 227610j, Columbus, Ohio, US; & DD-A-230 533 (VEB CHEMISCHE WERKE BUNA) 04-12-1985 --- | 1 | C 08 F   8/28<br>C 08 F   8/12<br>C 07 C   47/04 |
| D,A | US-A-4 247 487  (J.S. PERCY)<br>* Claims 1-7 *<br>--- | 1 | |
| D,A | US-A-4 085 079  (R.C. KMETZ)<br>* Claims 1-9 *<br>--- | 1 | |
| A | GB-A- 856 840  (WACKER-CHEMIE)<br>* Claims 1-11; example *<br>--- | 1 | |
| A | GB-A-1 199 652  (UNION CARBIDE CORP.)<br>* Claims 1-6 *<br>--- | 1 | |
| A | DE-C- 755 027  (SOCIETE NOBEL FRANCAISE, PARIS)<br>* Claim *<br>----- | 1 | TECHNICAL FIELDS SEARCHED (Int. Cl.4)<br><br>C 08 F |

The present search report has been drawn up for all claims

| Place of search | Date of completion of the search | Examiner |
|---|---|---|
| THE HAGUE | 26-08-1988 | PERMENTIER W.A. |